# EUROPEAN PATENT SPECIFICATION

(11) **EP 3 655 036 B1**
(45) Date of publication and mention of the grant of the patent: **04.09.2024**
(21) Application number: 18750053.3
(22) Date of filing: 19.07.2018
(51) Int. Cl.: A61K 31/198, A61K 45/06, A61K 9/00, A61P 25/00, A61P 25/24, A61P 25/28, A61P 43/00

(54) **COMPOSITIONS FOR TREATING STRESS-RELATED DISORDERS**
ZUSAMMENSETZUNGEN ZUR BEHANDLUNG VON STRESSBEDINGTEN STÖRUNGEN
COMPOSITIONS POUR LE TRAITEMENT DE TROUBLES LIÈS AU STRESS

(30) Priority: 19.07.2017 US 201715653837; 09.02.2018 US 201862628687 P
(43) Date of publication of application: 27.05.2020
(73) Proprietor: Hoffman Technologies, LLC, New York, New York 10022 (US)
(72) Inventor: HOFFMAN, Steven, Mahwah, NJ 07430 (US); ROTHMAN, John, Lebanon, NJ 07430 (US)
(74) Representative: Høiberg P/S
(86) International application number: PCT/US2018/042874
(87) International publication number: WO 2019/018633

(56) References cited:
- WO-A1-2012/123819
- WO-A1-2014/045023
- WO-A1-2014/078724
- US-A1- 2017 029 892
- JONEC V ET AL: "Effect of inhibitors of protein synthesis on the development of kindled seizures in rats", EXPERIMENTAL NEUROLOGY, ELSEVIER, AMSTERDAM, NL, vol. 66, no. 3, 1 December 1979 (1979-12-01), pages 524 - 532, XP022977096, ISSN: 0014-4886, [retrieved on 19791201], DOI: 10.1016/0014-4886(79)90199-7
- SQUIRE L R ET AL: "Tyrosine hydroxylase inhibition by cycloheximide and anisomycin is not responsible for their amnesic effect", BRAIN RESEARCH, ELSEVIER, AMSTERDAM, NL, vol. 82, no. 2, 27 December 1974 (1974-12-27), pages 241 - 248, XP024284427, ISSN: 0006-8993, [retrieved on 19741227], DOI: 10.1016/0006-8993(74)90601-5
- COHEN H ET AL: "Anisomycin, a Protein Synthesis Inhibitor, Disrupts Traumatic Memory Consolidation and Attenuates Posttraumatic Stress Response in Rats", BIOLOGICAL PSYCHIATRY, ELSEVIER SCIENCE, NEW YORK, NY; US, vol. 60, no. 7, 1 October 2006 (2006-10-01), pages 767 - 776, XP027916943, ISSN: 0006-3223, [retrieved on 20061001]
- SOPHIE A. GEORGE ET AL: "Altered locus coeruleus-norepinephrine function following single prolonged stress", EUROPEAN JOURNAL OF NEUROSCIENCE., vol. 37, no. 6, 20 December 2012 (2012-12-20), GB, pages 901 - 909, XP055510850, ISSN: 0953-816X, DOI: 10.1111/ejn.12095

## Description

### TECHNICAL FIELD

The present inventions relate generally to compositions for use in methods for the treatment of stress-related disorders, including post-traumatic stress disorder.

### BACKGROUND

The American Psychiatric Associate defines post-traumatic stress disorder ("PTSD") as a psychiatric disorder that can occur in people who have experienced or witnessed a traumatic event such as a natural disaster, a serious accident, a terrorist act, war/combat, rape or other violent personal assault. PTSD, once called shell shock or battle fatigue syndrome, typically is a lasting consequence of traumatic ordeals that cause intense fear, helplessness, or horror, such as a sexual or physical assault, the unexpected death of a loved one, an accident, war, or natural disaster. Families of victims can also develop PTSD, as can emergency personnel and rescue workers.

Most people who experience a traumatic event will have reactions that include shock, anger, nervousness, fear, and even guilt. For most people, these feelings they go away over time. But for a person with PTSD, these feelings continue and even increase, becoming so strong that they keep the person from living a normal life.

Those suffering from PTSD typically have abnormal levels of stress hormones. Studies have shown that individuals with PTSD have lower levels of cortisol than those who do not have PTSD and higher than average levels of epinephrine and norepinephrine.

There exists a need for improved compositions and methods for treating PTSD.

WO 2012123819 describes a nasal delivery device for and method of delivering a substance, preferably comprising oxytocin, non-peptide agonists thereof and antagonists thereof, preferably as one of a liquid, as a suspension or solution, or a powder, to the nasal airway of a subject, preferably the posterior region of the nasal airway, and preferably the upper posterior region of the nasal airway which includes the olfactory bulb and the trigeminal nerve, and preferably in the treatment of neurological conditions and disorders.

US 2017029892 describes methods and compositions to treat neuropsychiatric disorders post-traumatic stress disorder (PTSD). In particular, described therein are angiotensin receptor blockers (ARBs), and in particular the combination of one or more ARB (such as telmisartan) and an agent that enhances the delivery of the ARB across the blood-brain barrier (such as minocycline). PTSD may be treated using a combination of telmisartan and minocycline at levels of each that are, by themselves, infective to treat PTSD. Also described therein are methods for treating PTSD by first identifying patents for whom the use of an ARB treatment would be effective, by determining that patient has a dysfunction in their angiotensin converting enzyme and/or other genes in the autonomic arousal axis.

Cohen, H. et al., Biological Psychiatry 2006 describes that Anisomycin, a protein synthesis inhibitor, disrupts traumatic memory consolidation and attenuates posttraumatic stress response in rats.

Sophie A. George et al., Biological Psychiatry 2006 describes altered locus coeruleus-norepinephrine function following single prolonged stress.

WO 2014045023 describes treatment or prevention of post-traumatic stress disorder (PTSD). In particular, the document relates to an isolated Mycobacterium, for use in the prevention of PTSD and the symptoms associated with such a disorder. Also provided are methods of improving resilience in a subject by administering a therapeutically effective amount of isolated Mycobacterium.

WO 2014078724 describes methods for treating post traumatic stress disorder (PTSD) in a subject. The methods include administering a therapeutically effective amount of a neurotoxin to a corrugator supercilli and/or a procerus muscle of the subject to cause paralysis of the corrugator supercilli and/or a procerus muscle in the subject, thereby treating PTSD. The neurotoxin can be Botulinum toxin A, such as at a dose of about 20 to about 50 units of Botulinum toxin A.

Jonec, V. et al., Experimental Neurology 1979, describes the effect of inhibitors of protein synthesis on the development of kindled seizures in rats.

Squire, L.R et al., Brain Research 1974, describes that tyrosine hydroxylase inhibition by cycloheximide and anisomycin is not responsible for their amnesic effect.

### SUMMARY

The present invention provides compositions for use for treating PTSD and other stress-related disorders in a subject in need thereof. In certain embodiments, the composition for use comprises an effective amount of a tyrosine hydroxylase inhibitor. In other embodiments, the invention provides a composition for use comprising an effective amount of one or more stress modulators such as vasopressin, vasopressin derivatives, acetylcholine inducers, γ-aminobutyric acid (GABA), and other agents known to be useful in the treatment of anxiety or agitation.

The invention is defined by the claims. Any subject matter falling outside the scope of the claims is provided for information purposes only".

The references to methods of treatment in this description are to be interpreted as references to the compounds, pharmaceutical compositions and medicaments of the present invention for use in a method for the treatment of the human (or animal) body by therapy (or for diagnosis.

In an aspect, the present disclosure is directed to a composition comprising at least one tyrosine hydroxylase inhibitor for use in treating post-traumatic stress disorder in a subject in need thereof wherein said at least one tyrosine hydroxylase inhibitor is one or more of methyl (2R)-2-amino-3-(2-chloro-4 hydroxyphenyl) propanoate, D-tyrosine ethyl ester hydrochloride, methyl (2R)-2-amino-3-(2,6-dichloro-3,4-dimethoxyphenyl) propanoate H-D-Tyr(TBU)-allyl ester HCl, methyl (2R)-2-amino-3-(3-chloro-4,5-dimethoxyphenyl) propanoate, methyl (2R)-2-amino-3-(2-chloro-3-hydroxy-4-methoxyphenyl) propanoate, methyl (2R)-2-amino-3-(4-[(2-chloro-6-fluorophenyl) methoxy] phenyl) propanoate, methyl (2R)-2-amino-3-(2-chloro-3,4-dimethoxyphenyl) propanoate, methyl (2R)-2-amino-3-(3-chloro-5-fluoro-4-hydroxyphenyl) propanoate, diethyl 2-(acetylamino)-2-(4-[(2-chloro-6-fluorobenzyl) oxy] benzyl malonate, methyl (2R)-2-amino-3-(3-chloro-4-methoxyphenyl)propanoate, methyl (2R)-2-amino-3-(3-chloro-4-hydroxy-5-methoxyphenyl) propanoate, methyl (2R)-2-amino-3-(2,6-dichloro-3-hydroxy-4-methoxyphenyl) propanoate, methyl (2R)-2-amino-3-(3-chloro-4-hydroxyphenyl) propanoate, H-DL-tyr-OMe HCl, H-3,5-diiodo-tyr-OMe HCl, H-D-3,5-diiodo-tyr-OMe HCl, H-D-tyr-OMe HCl, D-tyrosine methyl ester hydrochloride, D-tyrosine-OMe HCl, methyl D-tyrosinate hydrochloride, (2R)-2-amino-3-(4-hydroxyphenyl) propionic acid, (2R)-2-amino-3-(4-hydroxyphenyl) methyl ester hydrochloride, methyl (2R)-2-amino-3-(4-hydroxyphenyl) propanoate hydrochloride, methyl (2R)-2-azanyl-3-(4-hydroxyphenyl) propanoate hydrochloride, 3-chloro-L-tyrosine, 3-nitro-L-tyrosine, 3-nitro-L-tyrosine ethyl ester hydrochloride, DL-*m*-tyrosine, DL-*o*-tyrosine, Boc-Tyr(3,5-I₂)-OSu, Fmoc-tyr(3-NO₂)-OH, α-methyl-L-tyrosine, α-methyl-D-tyrosine, and α-methyl-DL-tyrosine

### DETAILED DESCRIPTION OF ILLUSTRATIVE EMBODIMENTS

The present subject matter may be understood more readily by reference to the following detailed description which forms a part of this disclosure.

Unless otherwise defined herein, scientific and technical terms used in connection with the present application shall have the meanings that are commonly understood by those of ordinary skill in the art. Further, unless otherwise required by context, singular terms shall include pluralities and plural terms shall include the singular.

As employed above and throughout the disclosure, the following terms and abbreviations, unless otherwise indicated, shall be understood to have the following meanings.

In the present disclosure the singular forms "a," "an," and "the" include the plural reference, and reference to a particular numerical value includes at least that particular value, unless the context clearly indicates otherwise. Thus, *e.g.,* a reference to "a compound" is a reference to one or more of such compounds and equivalents thereof known to those skilled in the art, and so forth. The term "plurality", as used herein, means more than one. When a range of values is expressed, another embodiment incudes from the one particular and/or to the other particular value. Similarly, when values are expressed as approximations, by use of the antecedent "about," it is understood that the particular value forms another embodiment. All ranges are inclusive and combinable.

As used herein, the terms "component," "composition," "composition of compounds," "compound," "drug," "pharmacologically active agent," "active agent," "therapeutic," "therapy," "treatment," or "medicament" are used interchangeably herein to refer to a compound or compounds or composition of matter which, when administered to a subject (human or animal) induces a desired pharmacological and/or physiologic effect by local and/or systemic action.

As used herein, the terms "treatment" or "therapy" (as well as different forms thereof) include preventative (*e.g*., prophylactic), curative or palliative treatment. As used herein, the term "treating" includes alleviating or reducing at least one adverse or negative effect or symptom of a condition, disease or disorder. Beneficial or desired clinical results as a result of treatment or therapy include alleviation of symptoms, diminishment of the extent of a disease or condition, stabilization of a disease or condition (i.e., where the disease or condition does not worsen), delay or slowing of the progression of a disease or condition, amelioration or palliation of the disease or condition, and remission (whether partial or total) of the disease or condition, whether detectable or undetectable. Those in need of treatment include those already with the disease or condition as well as those prone to having the disease or condition or those in which the disease or condition is to be prevented.

The term "alkyl" is used herein to refer to both straight- and branched-chain saturated aliphatic hydrocarbon groups. In some embodiments, an alkyl group as 1 to about 6 carbon atoms. In other embodiments, an alkyl group has 1 to about 4 carbon atoms, i.e., a "lower alkyl".

The term "alkenyl" refers to an alkyl group containing at least one double bond. In some embodiments, an alkenyl group contains at least one double bond. In further embodiments, an alkenyl group contains one or two double bonds. In yet further embodiments, the alkenyl group contains one double bond. In other embodiments, an alkenyl group has 1 to about 6 carbon atoms. In other embodiments, an alkenyl group has 1 to about 4 carbon atoms, *i.e.*, a lower "alkenyl".

The term "halogen" as used herein refers to Cl, Br, F, or I groups.

The diseases or disorders treated as described herein include, post-traumatic stress disorder *e.g.,* PTSD. According to the National Center for PTSD, there are five main types of post-traumatic stress disorder: normal stress response, acute stress disorder, uncomplicated PTSD, comorbid PTSD and complex PTSD. For the purposes of this specification, PTSD may include any of the known types and subtypes. In some embodiments, the PTSD is a normal stress response PTSD. In further embodiments, the PTSD is an acute stress disorder PTSD. In other embodiments, the PTSD is uncomplicated PTSD. In yet further embodiments, the PTSD is comorbid PTSD. In still other embodiments, the PTSD is complex PTSD.

With respect to PTSD, *e.g.,* the adverse or negative effect or symptoms can include any of those that are the subject of the diagnostic criteria specified for PTSD in the American Psychiatric Association's Diagnostic and Statistical Manual, Fifth Edition (DSM-5, DSM-V), including: at least one of intrusive thoughts, nightmares, flashbacks, emotional distress after exposure to traumatic reminders, and physical reactivity after exposure to traumatic reminders; at least one of trauma-related thoughts or feelings and trauma-related reminders; at least two of inability to recall key features of the trauma, overly negative thoughts and assumptions about oneself or the world, exaggerated blame of self or others for causing the trauma, negative affect, decreased interest in activities, feeling isolated, and difficulty experiencing positive affect; and at least two of irritability or aggression, risky or destructive behavior, hypervigilance, heightened startle reaction, difficulty concentrating, and difficulty sleeping. Assessment of PTSD symptoms, or any of the symptoms of the present disclosure, can be performed using methods known in the art.

As used above and throughout the disclosure the term "effective amount" refers to an amount effective, at dosages, and for periods of time necessary, to achieve the desired result with respect to the treatment of the relevant disorder, condition, or side effect. It will be appreciated that the effective amount of components of the present invention will vary from patient to patient not only with respect to the particular compound, component or composition selected, the route of administration, and the ability of the components to elicit a desired result in the individual, but also with respect to factors such as the disease state or severity of the condition to be alleviated, hormone levels, age, sex, weight of the individual, the state of being of the patient, the severity of the pathological condition being treated, concurrent medication, special diets then being followed by the particular patient, means of administration, target site, physiological state of the patient, whether the patient is human or an animal, or whether treatment is prophylactic or therapeutic and other factors which those skilled in the art will recognize, with the appropriate dosage being at the discretion of the attending physician. Dosage regimes may be adjusted to provide improved therapeutic response using routine methods known to those of skill in the art to optimize safety and efficacy. An effective amount is also one in which any toxic or detrimental effects of the components are outweighed by the therapeutically beneficial effects.

"Pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

Within the present invention, the disclosed compounds may be prepared in the form of pharmaceutically acceptable salts. "Pharmaceutically acceptable salts" refer to derivatives of the disclosed compounds wherein the parent compound is modified by making acid or base salts thereof. Examples of pharmaceutically acceptable salts include mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, *e.g*., from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. These physiologically acceptable salts are prepared by methods known in the art, *e.g.,* by dissolving the free amine bases with an excess of the acid in aqueous alcohol, or neutralizing a free carboxylic acid with an alkali metal base such as a hydroxide, or with an amine.

Compounds described herein can be prepared in alternate forms. For example, many amino-containing compounds can be used or prepared as an acid addition salt. Often such salts improve isolation and handling properties of the compound. For example, depending on the reagents, reaction conditions and the like, compounds as described herein can be used or prepared, *e.g.,* as their hydrochloride or tosylate salts. Isomorphic crystalline forms, all chiral and racemic forms, N-oxide, hydrates, solvates, and acid salt hydrates, are also contemplated to be within the scope of the present invention.

Certain acidic or basic compounds of the present invention may exist as zwitterions. All forms of the compounds, including free acid, free base and zwitterions, are contemplated to be within the scope of the present invention. It is well known in the art that compounds containing both amino and carboxy groups often exist in equilibrium with their zwitterionic forms. Thus, any of the compounds described herein that contain, *e.g*., both amino and carboxy groups, also include reference to their corresponding zwitterions.

The term "stereoisomers" refers to compounds that have identical chemical constitution, but differ as regards the arrangement of the atoms or groups in space. The term "enantiomers" refers to stereoisomers that are mirror images of each other that are non-superimposable.

The term "administering" means either directly administering a compound or composition of the present invention, or administering a prodrug, derivative or analog which will form an equivalent amount of the active compound or substance within the body.

The terms "subject," "individual," and "patient" are used interchangeably herein, and refer to an animal, *e.g.,* a human, to whom treatment, including prophylactic treatment, with the pharmaceutical composition according to the present invention, is provided. The term "subject" as used herein refers to human and non-human animals. The terms "non-human animals" and "non-human mammals" are used interchangeably herein and include all vertebrates, *e.g*., mammals, such as non-human primates, (particularly higher primates), sheep, dog, rodent, (*e.g*. mouse or rat), guinea pig, goat, pig, cat, rabbits, cows, horses and non-mammals such as reptiles, amphibians, chickens, and turkeys. In some embodiments, the patient is a human. In other embodiments, the patient is a non-human mammal. In further embodiments, the patient is a non-human transgenic mammal.

The term "inhibitor" as used herein includes compounds that inhibit the expression or activity of a protein, polypeptide or enzyme and does not necessarily mean complete inhibition of expression and/or activity. Rather, the inhibition includes inhibition of the expression and/or activity of a protein, polypeptide or enzyme to an extent, and for a time, sufficient to produce the desired effect.

While not intending to be bound by any particular mechanism of operation, it is believed that the composition for use in methods of the present invention treat PTSD and other stress-related disorders by decreasing the amount of catecholamines secreted into the bloodstream.

Compositions for use in methods of treating intestinal PTSD and other stress-related disorders in a subject are provided. Such methods can include administering to a subject in need thereof an effective amount of a tyrosine hydroxylase inhibitor. Other such methods include administering to a subject in need thereof an effective amount of tyrosine hydroxylase inhibitor and an effective amount of one or more additional therapeutic agents, for example, one or more additional "stress modulators."

Following the administration of compositions for use in the methods disclosed herein, at least one of the following symptoms will be reduced in duration and/or severity: intrusive thoughts, nightmares, flashbacks, emotional distress after exposure to traumatic reminders, physical reactivity after exposure to traumatic reminders, trauma-related thoughts or feelings, trauma-related reminders, inability to recall key features of the trauma, overly negative thoughts and assumptions about oneself or the world, exaggerated blame of self or others for causing the trauma, negative affect, decreased interest in activities, feeling isolated, difficulty experiencing positive affect, irritability or aggression, risky or destructive behavior, hypervigilance, heightened startle reaction, difficulty concentrating, and difficulty sleeping.

This tyrosine hydroxylase inhibitor and the additional therapeutic agent (*e.g*., stress modulator) can be administered simultaneously, separately, or sequentially. In some embodiments, the tyrosine hydroxylase inhibitor and additional agent (*e.g*., stress modulator) are administered simultaneously. In further embodiments, the tyrosine hydroxylase inhibitor and the additional agent (*e.g*., stress modulator) are administered separately. In other embodiments, the tyrosine hydroxylase inhibitor and the additional agent (*e.g*., stress modulator) are administered sequentially. In some aspects, the additional agent (*e.g*., stress modulator) is administered at bedtime.

Administration of the tyrosine hydroxylase inhibitor or the tyrosine hydroxylase inhibitor and the additional agent (*e.g*., stress modulator) can be through various routes, including orally, nasally subcutaneously, intravenously, intramuscularly, transdermally, vaginally, rectally or in any combination thereof. Transdermal administration can be effected using, *e.g.,* oleic acid, 1-methyl-2-pyrrolidone, dodecylnonaoxyethylene glycol monoether.

In other suitable embodiments of the invention the tyrosine hydroxylase inhibitor and the additional agent (*e.g*., stress modulator) are administered daily or during a cycle consisting of five to seven days of administering the tyrosine hydroxylase inhibitor and the additional agent (*e.g*., stress modulator), and one to two days of not administering the tyrosine hydroxylase inhibitor and the stress modulator. In some embodiments, the tyrosine hydroxylase inhibitor is administered daily. In other embodiments, the tyrosine hydroxylase inhibitor and additional agent (*e.g*., stress modulator) are administered daily. In some suitable embodiments of the invention, at least six of said cycles of administration are performed. In some suitable embodiments, the tyrosine hydroxylase inhibitor and additional agent (*e.g*., stress modulator) are given in divided doses. In other embodiments, the tyrosine hydroxylase inhibitor is administered in one, two, three, or four doses, each day.

In some suitable embodiments of the invention, about 25 to about 500 mg of the tyrosine hydroxylase inhibitor is administered either as a single dose or in divided doses. In some aspects, the dosage of the tyrosine hydroxylase inhibitor is about 1 mg to about 4 g. In other aspects, the dosage of the tyrosine hydroxylase inhibitor is about 3 mg to about 1000 mg. In further aspects, the dosage of the tyrosine hydroxylase inhibitor is about 5 mg to about 500 mg, about 5 to about 100 mg, about 10 to about 100 mg, about 10 to about 100 mg, about 10 to about 90 mg, about 10 to about 80 mg, about 10 to about 70 mg, about 10 to about 60 mg, about 10 to about 50 mg, about 10 to about 40 mg, about 10 to about 30 mg, about 10 to about 25 mg, or about 10 to about 20 mg. In some suitable embodiments, the dosage of the tyrosine hydroxylase inhibitor is about 25 mg, about 10 mg, about 15 mg, about 20 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg. In further aspects, the dosage of the tyrosine hydroxylase inhibitor is about 60 mg. In other aspects, the dosage of the tyrosine hydroxylase inhibitor is about 60 mg for oral administration. In still further aspects, the dosage of the tyrosine hydroxylase inhibitor is about 0.25 mL of a 2 mg/mL suspension. In yet other aspects, the dosage of the tyrosine hydroxylase inhibitor is about 0.25 mL of a 2 mg/mL suspension for subcutaneous administration.

A preferred tyrosine hydroxylase inhibitor is well known in the art and fully described in, e.g., U.S. Patent Application Publication Nos. 2015/0290279, 2015/0216827, 2015/0111937, 2015/0111878, 2013/0184214, and 20130183263; U.S. Patent Nos. 8,481,498, 9,308,188, and 9,326,962; and International Patent Application Publication No. WO-2015/061328. Any suitable tyrosine hydroxylase inhibitor, known to one of skilled in the art, can be used.

In certain embodiments, the tyrosine hydroxylase inhibitor is a tyrosine derivative. The tyrosine derivative can be capable of existing in different isomeric forms, including stereoisomers and enantiomers. The tyrosine derivative can, *e.g.,* exist in L-form or D-form. The tyrosine derivative can, *e.g.,* also exist in a racemic form. Representative tyrosine derivatives include one or more of methyl (2R)-2-amino-3-(2-chloro-4 hydroxyphenyl) propanoate, D-tyrosine ethyl ester hydrochloride, methyl (2R)-2-amino-3-(2,6-dichloro-3,4-dimethoxyphenyl) propanoate H-D-Tyr(TBU)-allyl ester HCl, methyl (2R)-2-amino-3-(3-chloro-4,5-dimethoxyphenyl) propanoate, methyl (2R)-2-amino-3-(2-chloro-3-hydroxy-4-methoxyphenyl) propanoate, methyl(2R)-2-amino-3-(4-[(2-chloro-6-fluorophenyl) methoxy] phenyl) propanoate, methyl (2R)-2-amino-3-(2-chloro-3,4-dimethoxyphenyl) propanoate, methyl (2R)-2-amino-3-(3-chloro-5-fluoro-4-hydroxyphenyl) propanoate, diethyl 2-(acetylamino)-2-(4-[(2-chloro-6-fluorobenzyl) oxy] benzyl malonate, methyl (2R)-2-amino-3-(3-chloro-4-methoxyphenyl)propanoate, methyl (2R)-2-amino-3-(3-chloro-4-hydroxy-5-methoxyphenyl) propanoate, methyl (2R)-2-amino-3-(2,6-dichloro-3-hydroxy-4-methoxyphenyl) propanoate, methyl (2R)-2-amino-3-(3-chloro-4-hydroxyphenyl) propanoate, H-DL-tyr-OMe HCl, H-3,5-diiodo-tyr-OMe HCl, H-D-3,5-diiodo-tyr-OMe HCl, H-D-tyr-OMe HCl, D-tyrosine methyl ester hydrochloride, D-tyrosine-OMe HCl, methyl D-tyrosinate hydrochloride, (2R)-2-amino-3-(4-hydroxyphenyl) propionic acid, (2R)-2-amino-3-(4-hydroxyphenyl) methyl ester hydrochloride, methyl (2R)-2-amino-3-(4-hydroxyphenyl) propanoate hydrochloride, methyl (2R)-2-azanyl-3-(4-hydroxyphenyl) propanoate hydrochloride, 3-chloro-L-tyrosine, 3-nitro-L-tyrosine, 3-nitro-L-tyrosine ethyl ester hydrochloride, DL-*m*-tyrosine, DL-*o*-tyrosine, Boc-Tyr(3,5-I₂)-OSu, Fmoc-tyr(3-NO₂)-OH, and α-methyl-DL-tyrosine. In certain embodiments of the invention, the tyrosine derivative is α-methyl-L-tyrosine as shown below:

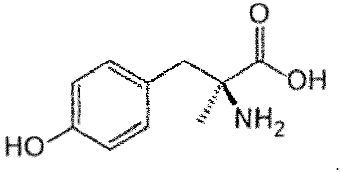

In other embodiments, the tyrosine derivative is α-methyl-D-tyrosine. In other embodiments, the tyrosine derivative is α-methyl-DL-tyrosine in a racemic form as shown below:

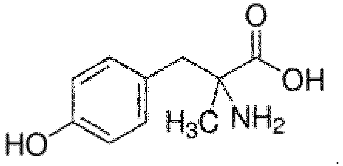

α-Methyl-DL-tyrosine is also referred herein as DNP-01 or L1-79 or AMPT or α-methyl-para-tyrosine. In other words, the alternative names of α-methyl-DL-tyrosine include, *e.g.,* DNP-01, L1-79, AMPT, and α-methyl-para-tyrosine.

In some embodiments, the tyrosine derivative is a structural variant of α-methyl-L-tyrosine or α-methyl-DL-tyrosine. Structural variants of α-methyl-L-tyrosine or α-methyl-DL-tyrosine are well known in the art and fully described in, *e.g.,* U.S. Patent No. 4,160,835.

In certain methods of the invention, 60 mg of the tyrosine derivative is administered orally and 0.25 mL of a 2 mg/mL suspension of the tyrosine derivative is administered subcutaneously. Preferably, 50-1500 mg of α-methyl-DL-tyrosine is administered daily.

In other aspects, the disclosure describes administering a therapeutically effective amount of one or more additional therapeutic agents, for example, one or more "stress modulators," useful in the treatment of PTSD. Representative therapeutic agents. *e.g*., stress modulators, include vasopressin, vasopressin derivatives, acetylcholine inducers, glucocorticoids, cannabinoids, neuromodulators, or other agents known to be useful in the treatment of anxiety or agitation, including drugs in the benzodiazepine class or antidepressants.

Vasopressin derivatives include 1-deamino-8-D-arginine vasopressin (DDAVP), 1-deamino-4-valin-8-D-arginine vasopressin (DVDAVP) and 8-arginine vasopressin (AVP).

Acetylcholine inducers include both direct and indirect acetylcholine agonists such as melanotans, vasopressin, desmopressin, bethanechol, carbachol, cevimeline, pilocarpine, ambenomium, demecarium, donezepil, edrophonium, galantamine, neostigmine, physostigmine, pyridostigmine, rivastigmine, tacrine, and echotiophate (including salts thereof).

Representative benzodiazepine drugs include clorazepate, diazepam, flurazepam, halazepam, prazepam, lorazepam, lormetazepam, oxazepam, temazepam, clonazepam, flunitrazepam, nimetazepam, nitrazepam, adinazolam, alprazolam, estazolam, triazolam, climazolam, loprazolam, and midazolam.

In other embodiments, the one or more additional therapeutic agents, *e.g*., stress modulators, is an antidepressant, *e.g*., selective serotonin reuptake inhibitors (SSRI), a serotonin-norepinephrine reuptake inhibitors (SNRI), or tricyclic antidepressants. Additional examples of antidepressants include sertraline (Zoloft), fluoxetine, paroxetine (Paxil), venlafaxine, or prazosin. Representative SSRIs include citalopram, fluvoxamine, escitalopram, paroxetine, sertraline, and fluoxetine (including salts thereof).

In some embodiments, a therapeutically effective concentration of other neuromodulators may be added along with a tyrosine hydroxylase inhibitor. Examples of such neuromodulators include vasopressin or analogs such as desmopressin, and agents which stimulate effective concentrations of acetylcholine or gamma-aminobutyric acid (GABA).

The dosage of the one or more stress modulator may include a therapeutically effective or clinically acceptable amount. In another example, the dosage of the one or more additional therapeutic agents, *e.g*., "stress modulators," is an amount that complements with or enhances the effect of a tyrosine hydroxylase inhibitor described herein.

In some embodiments, the administration may be by any suitable route known to one of skilled in the art. Administration of pharmaceutically active molecules, or compositions containing one or more pharmaceutically active molecules, can be through various routes, including orally, nasally, parenterally (e.g., subcutaneously, intravenously, intramedullary, intraarticularly, intramuscularly, or intraperitoneally), topically, transdermally, vaginally, rectally or in any combination thereof. Transdermal administration can be effected using, *e.g.,* oleic acid, 1-methyl-2-pyrrolidone, dodecylnonaoxyethylene glycol monoether. In some embodiments, composition is administered orally (e.g., in capsules, suspensions, or tablets. In other embodiments, the composition is administered parenterally (e.g., intravenous, subcutaneous, intraperitoneal, intramuscular). In further embodiments, the composition is administered by intravenous infusion or injection. In yet other embodiments, the composition is administered intramuscularly or subcutaneously. In one embodiment, tyrosine hydroxylase inhibitor is co-administered with another PTSD treating agent. In another embodiment, tyrosine hydroxylase inhibitor is administered independently from the administration of another PTSD treating agent. In one embodiment, tyrosine hydroxylase inhibitor is administered first, followed by the administration of another PTSD treating agent. In another embodiment, said another PTSD treating agent is administered first, followed by the administration of said tyrosine hydroxylase inhibitor.

The administration of the tyrosine hydroxylase inhibitor with another PTSD treating agent and/or other treatments may occur simultaneously, or separately, via the same or different route, at the same or different times. Dosage regimens may be adjusted to provide the optimum desired response (e.g., a therapeutic or prophylactic response).

### II. Compositions

Further provided are pharmaceutical compositions comprising compounds described herein and one or more pharmaceutically acceptable carriers. As used herein, a "composition" refers to any composition that contains a pharmaceutically effective amount of one or more active ingredients (e.g., a tyrosine hydroxylase inhibitor, another PTSD treating agent, or a combination thereof). "Pharmaceutically acceptable carriers" include any excipient which is nontoxic to the cell or mammal being exposed thereto at the dosages and concentrations employed. The pharmaceutical composition may include one or additional therapeutic agents. Physiologically acceptable salt forms and standard pharmaceutical formulation techniques are well known to persons skilled in the art. See, *e.g*., Remington's Pharmaceutical Sciences, Mack Publishing Co.

Pharmaceutically acceptable carriers include solvents, dispersion media, buffers, coatings, antibacterial and antifungal agents, wetting agents, preservatives, buggers, chelating agents, antioxidants, isotonic agents and absorption delaying agents.

Pharmaceutically acceptable carriers include water; saline; phosphate buffered saline; dextrose; glycerol; alcohols such as ethanol and isopropanol; phosphate, citrate and other organic acids; ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; EDTA; salt forming counterions such as sodium; and/or nonionic surfactants such as TWEEN, polyethylene glycol (PEG), and PLURONICS; isotonic agents such as sugars, polyalcohols such as mannitol and sorbitol, and sodium chloride; as well as combinations thereof.

The pharmaceutical compositions may be formulated in a variety of ways, including, *e.g*., solid, semi-solid, and liquid dosage forms, such as tablets, pills, powders, liquid solutions (e.g., injectable and infusible solutions), dispersions or suspensions, liposomes and suppositories. In some embodiments, the compositions are in the form of injectable or infusible solutions. The composition is in a form suitable for oral, intravenous, intraarterial, intramuscular, subcutaneous, parenteral, transmucosal, topical, or transdermal administration. The composition may be formulated as an immediate, controlled, extended or delayed release composition.

Preparations for administration may also include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Pharmaceutically acceptable carriers include 0.01-0.1M and preferably 0.05M phosphate buffer or 0.8% saline. Other common parenteral vehicles include sodium phosphate solutions, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present such as, e.g., antimicrobials, antioxidants, chelating agents, and inert gases and the like.

The pharmaceutical compositions also are suitable for injectable use include sterile aqueous solutions (where water soluble) or dispersions and sterile powders for the extemporaneous preparation of sterile injectable solutions or dispersions. In such cases, the composition must be sterile and should be fluid to the extent that easy syringability exists. It should be stable under the conditions of manufacture and storage and will preferably be preserved against the contaminating action of microorganisms, such as bacteria and fungi. The carrier can be a solvent or dispersion medium containing, *e.g*., water, ethanol, polyol (e.g., glycerol, propylene glycol, and liquid polyethylene glycol, and the like), and suitable mixtures thereof The proper fluidity can be maintained, *e.g*., by the use of a coating such as lecithin, by the maintenance of the required particle size in the case of dispersion and by the use of surfactants. Suitable formulations for use in the therapeutic methods disclosed herein are described in Remington's Pharmaceutical Sciences, Mack Publishing Co., 16th ed. (1980).

In some embodiments, the composition includes isotonic agents, *e.g*., sugars, polyalcohols, such as mannitol, sorbitol, or sodium chloride. Prolonged absorption of the injectable compositions can be brought about by including in the composition an agent which delays absorption, *e.g*., aluminum monostearate and gelatin.

Sterile solutions can be prepared by incorporating the tyrosine hydroxylase inhibitor, by itself or in combination with other active agents, in the required amount in an appropriate solvent with one or a combination of ingredients enumerated herein, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, one method of preparation is vacuum drying and freeze-drying, which yields a powder of an active ingredient plus any additional desired ingredient from a previously sterile-filtered solution thereof. The preparations for injections are processed, filled into containers such as ampoules, bags, bottles, syringes or vials, and sealed under aseptic conditions according to methods known in the art. Further, the preparations may be packaged and sold in the form of a kit such as those described in US Patent Application Publication No. 2002/0102208. Such articles of manufacture will preferably have labels or package inserts indicating that the associated compositions are useful for treating a subject suffering from, or predisposed to PTSD.

The composition may be administered only once, or it may be administered multiple times. For multiple dosages, the composition may be, *e.g*., administered three times a day, twice a day, once a day, once every two days, twice a week, weekly, once every two weeks, or monthly.

In other embodiments, a single bolus may be administered. In further embodiments, several divided doses may be administered over time. In yet other embodiments, a dose may be proportionally reduced or increased as indicated by the exigencies of the therapeutic situation. Dosage unit form, as used herein, refers to physically discrete units suited as unitary dosages for treating mammalian subjects. Each unit may contain a predetermined quantity of active compound calculated to produce a desired therapeutic effect. In some embodiments, the dosage unit forms are dictated by and directly dependent on the unique characteristics of the active compound and the particular therapeutic or prophylactic effect to be achieved.

### III. Embodiments

In some aspects, a subject is administered a therapeutically effective amount of a first tyrosine hydroxylase inhibitor, *e.g*., α-methyl-DL-tyrosine in combination with a therapeutically effective amount of a second tyrosine hydroxylase inhibitor, *e.g*., α-methyl-L-tyrosine.

In other aspects, a subject is administered a therapeutically effective amount of one or more tyrosine hydroxylase inhibitors, *e.g*., α-methyl-DL-tyrosine, α-methyl-D-tyrosine, and/or α-methyl-L-tyrosine in combination with a therapeutically effective amount of another agent, such as a stress modulator, useful in the treatment of PTSD.

In yet further aspects, a composition comprises a tyrosine hydroxylase inhibitor, wherein said tyrosine hydroxylase inhibitor is present in an amount effective to treat PTSD, for example, effective to treat one or more symptoms of PTSD, in a subject. In some embodiments, the tyrosine hydroxylase inhibitor is α-methyl-DL-tyrosine. In other embodiments, the composition further comprises a therapeutically effective amount of another tyrosine hydroxylase inhibitor, *e.g*., metyrosine or α-methyl-L-tyrosine. In further embodiments, the composition further comprises a therapeutically effective amount of another agent, known to one of skilled in the art, useful in the treatment of PTSD, such as a stress modulator. In yet other embodiments, the tyrosine hydroxylase inhibitor is racemic α-methyl-DL-tyrosine.

In further aspects, the disclosure provides compositions comprising a tyrosine hydroxylase inhibitor, wherein said tyrosine hydroxylase inhibitor is present in an amount effective to treat a PTSD in a subject.

In other aspects, the disclosure provides compositions comprising a tyrosine hydroxylase inhibitor and a stress modulator useful in the treatment of PTSD, wherein said tyrosine hydroxylase inhibitor and said stress modulator are present in an amount effective to treat said PTSD in said subject.

### DNP-01

DNP-01 (also referred as L1-79) is D,L α-methyl-para-tyrosine and is abbreviated AMPT. α-methyl-para-tyrosine inhibits the activity of tyrosine hydroxylase (TH), which catalyzes the first transformation in catecholamine biosynthesis, i.e., the conversion of tyrosine to dihydroxyphenylalanine (DOPA) which is the rate limiting step in catecholamine synthesis. L α-methyl-para-tyrosine was approved by the FDA in 1979, is marketed under the name Demser^{®}, and is typically called metyrosine and abbreviated AMT.

α-Methyl-para-tyrosine is a tyrosine analog that competes competitively for TH and is excreted mostly unchanged in the urine. Demser was approved for presurgical use in the treatment of pheochromocytoma, a catecholamine producing tumor which when manipulated surgically releases pathologic levels of catecholamines into the circulation that can result in serious AE. Demser was approved to minimize this potentially serious complication, and to treat pheochromocytoma patients who were not qualified for surgery. It is approved for use in doses between 1 -4g/day in divided doses. AE associated with Demser include sedation that typically habituates but might persist at doses >2g/d, temporary changes in sleep, extrapyramidal signs including tremor at high doses, trismus and parkinsonism at high doses, dose dependent confusion that resolves with dose reduction, dose related diarrhea, and infrequent AE that include crystalluria, nausea and vomiting, and impotence.

### DNP-01 for the Treatment of PTSD

DNP-01 is an agent that has the ability to realign the sympathetic and parasympathetic arms of the autonomic nervous system and correct the imbalance manifest in PTSD. DNP-01 not only reduces sympathetic traffic acutely, but when administered chronically it enables the overstimulated sympathetics to repeat and resume normal function. From a cognitive perspective, the sympathetic nervous system participates in the regulation of mood, reward, dream states (including nightmares), memory and recall, circadian sleep patterns, anxiety and agitation, and many other symptoms expressed in PTSD, including metabolic changes. As presented herein, a stressor that overstimulates the sympathetic nervous system may result in the release of various growth factors that cause these tracts to enlarge, thus expanding their physiologic effect. These growth factors are then produced in response to the exaggerated sympathetic response and are necessary to sustain the newly evolved neural architecture. Reducing sympathetic outflow may enable these changes in the sympathetic system to regress to the normal, pre-stress levels.

DNP-01 has a therapeutic effect in the short and intermediate term of treatment of PTSD and may have a curative effect in the long run. DNP-01 inhibits the enzyme tyrosine hydroxylase, which is the first step, and the rate limiting step, in the synthesis of catecholamines in the body. By reducing the synthesis, and thus the availability, of these neurotransmitters DNP-01 reduces the release of the growth factors that support the imbalanced sympathetic nervous functions as well as the dopaminergic reward and cognitive functions in the brain. This results in a therapeutic mechanism of action that is fundamentally different from that seen with receptor blockers, as they do not inhibit the release of growth factors. In the short and intermediate term, reduced catecholamine release should improve the symptoms associated with PTSD in most patients. In the long term, reductions in the levels of catecholamine release that support the higher than normal levels of growth factors should enable the hypertrophic SNS to regress to a pre-trauma configuration, which should eliminate PTSD symptoms permanently.

For treatment of PTSD, a composition having a tyrosine hydroxylase inhibitor (e.g., α-methyl-DL tyrosine) can be orally administered to a patient in single or divided doses (e.g., three to five times a day). The total daily dose can range between 100 mg and 500 mg.

The composition can be administered for a period of six weeks, with one or two days off between weekly cycles. The PTSD symptoms can be monitored for all subjects biweekly through, for example evaluation under Structured Interview for PTSD (SI-PTSD; Davidson et al., J Nervous Mental Disease 177:336-41 (1989)), the Clinician Administered PTSD Scale (CAPS; Blake et al., Behavior Therapist 13:187-8 (1990)) or the Short Screening Scale for DSM-IV PTSD (Breslau et al., Am J Psychiatry 156:908-11 (1999)).

Overall, the above-noted treatment can be well tolerated by the patient, and responses can be documented for the treatment.

### IV. Examples

### Example 1

Patients are screened and the extent to which they meet the DSM-V criteria for PTSD is assessed. A subgroup of those satisfying the criteria are administered a treatment regimen that includes a tyrosine hydroxylase inhibitor (i.e., α-methyl-DL tyrosine) at dose of 200 mg three times daily. Another subgroup is administered a treatment regimen that further includes an acetylcholine inducer (e. g., a melanotan). GABA is optionally administered to both subgroups. Following each administration of the treatment regimen, changes in the extent to which the subjects satisfy the DSM-5 criteria are again assessed.

### Example 2

A 21 year old male patient presented symptoms of PTSD. The manifestation of his illness included poor eye contact, suspicion of people's intentions with no specific reason, disturbed sleep with many episodes of dreams compelling him to verbalize moans in a seemingly predictable manner, terrible overall sleep that was not restful, nightmares, fear of his own personal utility to the world, thoughts of suicide, an unemotional detachment from people and a constant overall depression.

His personal story included separation from his parents due to their incarceration, many times moving in his youth, foster care parents, and violence and bullying against him as a child by other children and notably by his substance abusing father. As a corrections officer, he also witnessed things, such as rape, that deeply disturbed him.

He was administered 300 mg of the drug (i.e., racemic α-methyl-DL-tyrosine) 2 times a day for 3 days. After the treatment, all symptoms subsided. He had restful sleep, eye contact normalized, he began and held conversations, his fears and nightmares subsided, and he personally commented that he was calmer and slept soundly for the first time in years. He also found emotion when watching a movie at an appropriate time and said it was the first time he cried since childhood.

He continued the drug for 28 days and even greater improvements were observable, like taking an interest in his appearance, engaging others in conversation, enhanced appetite, and smiling and laughing, being able to express his emotions and so forth.

## Claims

1. A composition comprising at least one tyrosine hydroxylase inhibitor for use in treating post-traumatic stress disorder in a subject in need thereof wherein said at least one tyrosine hydroxylase inhibitor is one or more of methyl (2R)-2-amino-3-(2-chloro-4 hydroxyphenyl) propanoate, D-tyrosine ethyl ester hydrochloride, methyl (2R)-2-amino-3-(2,6-dichloro-3,4-dimethoxyphenyl) propanoate H-D-Tyr(TBU)-allyl ester HCl, methyl (2R)-2-amino-3-(3-chloro-4,5-dimethoxyphenyl) propanoate, methyl (2R)-2-amino-3-(2-chloro-3-hydroxy-4-methoxyphenyl) propanoate, methyl (2R)-2-amino-3-(4-[(2-chloro-6-fluorophenyl) methoxy] phenyl) propanoate, methyl (2R)-2-amino-3-(2-chloro-3,4-dimethoxyphenyl) propanoate, methyl (2R)-2-amino-3-(3-chloro-5-fluoro-4-hydroxyphenyl) propanoate, diethyl 2-(acetylamino)-2-(4-[(2-chloro-6-fluorobenzyl) oxy] benzyl malonate, methyl (2R)-2-amino-3-(3-chloro-4-methoxyphenyl)propanoate, methyl (2R)-2-amino-3-(3-chloro-4-hydroxy-5-methoxyphenyl) propanoate, methyl (2R)-2-amino-3-(2,6-dichloro-3-hydroxy-4-methoxyphenyl) propanoate, methyl (2R)-2-amino-3-(3-chloro-4-hydroxyphenyl) propanoate, H-DL-tyr-OMe HCl, H-3,5-diiodo-tyr-OMe HCl, H-D-3,5-diiodo-tyr-OMe HCl, H-D-tyr-OMe HCl, D-tyrosine methyl ester hydrochloride, D-tyrosine-OMe HCl, methyl D-tyrosinate hydrochloride, (2R)-2-amino-3-(4-hydroxyphenyl) propionic acid, (2R)-2-amino-3-(4-hydroxyphenyl) methyl ester hydrochloride, methyl (2R)-2-amino-3-(4-hydroxyphenyl) propanoate hydrochloride, methyl (2R)-2-azanyl-3-(4-hydroxyphenyl) propanoate hydrochloride, 3-chloro-L-tyrosine, 3-nitro-L-tyrosine, 3-nitro-L-tyrosine ethyl ester hydrochloride, DL-*m*-tyrosine, DL-*o*-tyrosine, Boc-Tyr(3,5-I₂)-OSu, Fmoc-tyr(3-NO₂)-OH, α-methyl-L-tyrosine, α-methyl-D-tyrosine, and α-methyl-DL-tyrosine.

2. The composition for use according to claim 1, wherein said at least one tyrosine hydroxylase inhibitor is racemic α-methyl-DL-tyrosine.

3. The composition for use according to any one of the preceding claims, wherein said at least one tyrosine hydroxylase inhibitor is formulated for oral, nasal, subcutaneous, intravenous, topical, transdermal, vaginal, or rectal administration, or a combination thereof.

4. The composition for use according to any one of the preceding claims, comprising 50-1500 mg of the at least one tyrosine hydroxylase inhibitor.

5. The composition for use according to any one of the preceding claims, wherein said at least one tyrosine hydroxylase inhibitor is formulated in three divided doses.

6. The composition for use according to any one of the preceding claims, wherein said at least one tyrosine hydroxylase inhibitor is α-methyl-L-tyrosine (metyrosine) or α-methyl-D-tyrosine.

7. The composition for use according to any one of the preceding claims, further comprising one or more stress modulators, said stress modulator being an antidepressant, a vasopressin derivative, a neuromodulating agent, an acetylcholine inducer, a benzodiazepine, a glucocorticoid or a cannabinoid, for example, wherein the vasopressin derivative is vasopressin or desmopressin; wherein the neuromodulating agent is GABA, rivastigmine, or pilocarpine; wherein the antidepressant is a selective serotonin reuptake inhibitor (SSRI), a serotonin-norepinephrine reuptake inhibitor (SNRI), or a tricyclic antidepressant; or wherein the antidepressant is sertraline, fluoxetine, paroxetine, or venlafaxine.

8. The composition for use according to claim 7, wherein said vasopressin derivative is 1-deamino-8-D-arginine vasopressin (DDAVP), 1-deamino-4-valin-8-D-arginine vasopressin (DVDAVP) and 8-arginine vasopressin (AVP).

9. The composition for use according to claims 7 or 8, wherein said tyrosine hydroxylase inhibitor is racemic α-methyl-DL-tyrosine and wherein the stress modulator is desompressin and GABA.

10. The composition for use according to claim 9, comprising α-methyl-DL-tyrosine, desompressin, and GABA.

11. The composition for use according to any one of the preceding claims, wherein the treatment reduces at least one of the following: intrusive thoughts, nightmares, flashbacks, emotional distress after exposure to traumatic reminders, physical reactivity after exposure to traumatic reminders, trauma-related thoughts or feelings, trauma-related reminders, inability to recall key features of the trauma, overly negative thoughts and assumptions about oneself or the world, exaggerated blame of self or others for causing the trauma, negative affect, decreased interest in activities, feeling isolated, difficulty experiencing positive affect, irritability or aggression, risky or destructive behavior, hypervigilance, heightened startle reaction, difficulty concentrating, and difficulty sleeping.

12. The composition for use according to any one of the preceding claims, wherein said composition is used in combination with psychotherapy, cognitive behavioral therapy, eye movement desensitization and reprocessing (EMDR) therapy, interpersonal psychotherapy, physical therapy, play therapy, or a combination thereof.

## Patentansprüche

1. Zusammensetzung, die mindestens einen Tyrosinhydroxylase-Inhibitor umfasst, zur Verwendung bei der Behandlung einer posttraumatischen Belastungsstörung bei einer Person, der diese benötigt, wobei der mindestens eine Tyrosinhydroxylase-Inhibitor eines oder mehrere der Folgenden ist: Methyl-(2R)-2-amino-3-(2-chlor-4-hydroxyphenyl)propanoat, D-Tyrosinethylesterhydrochlorid, Methyl-(2R)-2-amino-3-(2,6-dichlor-3,4-dimethoxyphenyl)propanoat H-D-Tyr(TBU)-allylester HCl, Methyl-(2R)-2-amino-3-(3-chlor-4,5-dimethoxyphenyl)propanoat, Methyl-(2R)-2-amino-3-(2-chlor-3-hydroxy-4-methoxyphenyl)propanoat, Methyl-(2R)-2-amino-3-(4-[(2-chlor-6-fluorphenyl) methoxy] phenyl)propanoat, (2R)-2-Amino-3-(2-chlor-3,4-dimethoxyphenyl)propanoat, Methyl-(2R)-2-amino-3-(3-chlor-5-fluor-4-hydroxyphenyl)propanoat, Diethyl-2-(acetylamino)-2-(4-[(2-chlor-6-fluorbenzyl)oxy]benzylmalonat, Methyl-(2R)-2-amino-3-(3-chlor-4-methoxyphenyl)propanoat, Methyl-(2R)-2-amino-3-(3-chlor-4-hydroxy-5-methoxyphenyl)propanoat, Methyl-(2R)-2-amino-3-(2,6-dichlor-3-hydroxy-4-methoxyphenyl)propanoat, Methyl-(2R)-2-amino-3-(3-chlor-4-hydroxyphenyl)propanoat, H-DL-tyr-OMe HCl, H-3,5-Diiod-tyr-OMe HCl, H-D-3,5-Diiod-tyr-OMe HCl, H-D-tyr-OMe HCl, D-Tyrosinmethylesterhydrochlorid, D-Tyrosin-OMe HCl, Methyl-D-tyrosinathydrochlorid, (2R)-2-Amino-3-(4-hydroxyphenyl)propionsäure, (2R)-2-Amino-3-(4-hydroxyphenyl)methylesterhydrochlorid, Methyl-(2R)-2-amino-3-(4-hydroxyphenyl)propanoathydrochlorid, Methyl-(2R)-2-azanyl-3-(4-hydroxyphenyl)propanoathydrochlorid, 3-Chlor-L-tyrosin, 3-Nitro-L-tyrosin, 3-Nitro-L-tyrosinethylesterhydrochlorid, DL-m-Tyrosin, DL-o-Tyrosin, Boc-Tyr(3,5-I₂)-OSu, Fmoc-tyr(3-NO₂)-OH, α-Methyl-L-tyrosin, α-Methyl-D-tyrosin und α-Methyl-DL-tyrosin.

2. Zusammensetzung zur Verwendung nach Anspruch 1, wobei der mindestens eine Tyrosinhydroxylase-Inhibitor racemisches α-Methyl-DL-tyrosin ist.

3. Zusammensetzung zur Verwendung nach einem der vorstehenden Ansprüche, wobei der mindestens eine Tyrosinhydroxylase-Inhibitor zur oralen, nasalen, subkutanen, intravenösen, topischen, transdermalen, vaginalen oder rektalen Verabreichung oder einer Kombination davon formuliert ist.

4. Zusammensetzung zur Verwendung nach einem der vorstehenden Ansprüche, umfassend 50-1500 mg des mindestens einen Tyrosinhydroxylase-Inhibitors.

5. Zusammensetzung zur Verwendung nach einem der vorstehenden Ansprüche, wobei der mindestens eine Tyrosinhydroxylase-Inhibitor in drei geteilten Dosen formuliert ist.

6. Zusammensetzung zur Verwendung nach einem der vorstehenden Ansprüche, wobei der mindestens eine Tyrosinhydroxylase-Inhibitor α-Methyl-L-tyrosin (Metyrosin) oder α-Methyl-D-tyrosin ist.

7. Zusammensetzung zur Verwendung nach einem der vorstehenden Ansprüche, ferner umfassend einen oder mehrere Stressmodulatoren, wobei der Stressmodulator ein Antidepressivum, ein Vasopressin-Derivat, ein neuromodulierendes Mittel, ein Acetylcholin-Induktor, ein Benzodiazepin, ein Glukokortikoid oder ein Cannabinoid ist, beispielsweise wobei das Vasopressin-Derivat Vasopressin oder Desmopressin ist; wobei das neuromodulierende Mittel GABA, Rivastigmin oder Pilocarpin ist; wobei das Antidepressivum ein selektiver Serotonin-Wiederaufnahmehemmer (SSRI), ein Serotonin-Noradrenalin-Wiederaufnahmehemmer (SNRI) oder ein trizyklisches Antidepressivum ist; oder wobei das Antidepressivum Sertralin, Fluoxetin, Paroxetin oder Venlafaxin ist.

8. Zusammensetzung zur Verwendung nach Anspruch 7, wobei das Vasopressin-Derivat 1-Deamino-8-D-arginin-vasopressin (DDAVP), 1-Deamino-4-valin-8-D-arginin-vasopressin (DVDAVP) und 8-Arginin-vasopressin (AVP) ist.

9. Zusammensetzung zur Verwendung nach Anspruch 7 oder 8, wobei der Tyrosinhydroxylase-Inhibitor racemisches α-Methyl-DL-tyrosin ist und wobei der Stressmodulator Desompressin und GABA ist.

10. Zusammensetzung zur Verwendung nach Anspruch 9, umfassend α-Methyl-DL-tyrosin, Desompressin und GABA.

11. Zusammensetzung zur Verwendung nach einem der vorstehenden Ansprüche, wobei die Behandlung mindestens eines der Folgenden verringert: Zwangsgedanken, Albträume, Flashbacks, emotionale Belastung nach Konfrontation mit traumatischen Erinnerungen, körperliche Reaktivität nach Konfrontation mit traumatischen Erinnerungen, traumabezogene Gedanken oder Gefühle, traumabezogene Erinnerungen, Unfähigkeit, sich an Schlüsselmerkmale des Traumas zu erinnern, übermäßig negative Gedanken und Annahmen über sich selbst oder die Welt, übertriebene Schuldzuweisung an sich selbst oder andere für die Verursachung des Traumas, negative Affekte, vermindertes Interesse an Aktivitäten, Gefühl der Isolation, Schwierigkeiten beim Erleben positiver Affekte, Reizbarkeit oder Aggression, riskantes oder destruktives Verhalten, Hypervigilanz, erhöhte Schreckreaktion, Konzentrationsschwierigkeiten und Schlafstörungen.

12. Zusammensetzung zur Verwendung nach einem der vorstehenden Ansprüche, wobei die Zusammensetzung in Kombination mit Psychotherapie, kognitiver Verhaltenstherapie, Desensibilisierung und Aufarbeitung durch Augenbewegungen (EMDR), interpersoneller Psychotherapie, Physiotherapie, Spieltherapie oder einer Kombination davon verwendet wird.

## Revendications

1. Composition comprenant au moins un inhibiteur de tyrosine hydroxylase destinée à être utilisée dans le traitement du trouble de stress post-traumatique chez un sujet en ayant besoin, dans laquelle ledit au moins un inhibiteur de tyrosine hydroxylase est un ou plusieurs parmi le (2R)-2-amino-3-(2-chloro-4-hydroxyphényl)propanoate de méthyle, le chlorhydrate d'ester éthylique de D-tyrosine, le (2R)-2-amino-3-(2,6-dichloro-3,4-diméthoxyphényl)propanoate de méthyle, l'ester de H-D-Tyr(TBU)-allyle HCl, le (2R)-2-amino-3-(3-chloro-4,5-diméthoxyphényl)propanoate de méthyle, le (2R)-2-amino-3-(2-chloro-3-hydroxy-4-méthoxyphényl)propanoate de méthyle, le (2R)-2-amino-3-(4-[(2-chloro-6-fluorophényl)méthoxy]phényl)-propanoate de méthyle, le (2R)-2-amino-3-(2-chloro-3,4-diméthoxyphényl)propanoate de méthyle, le (2R)-2-amino-3-(3-chloro-5-fluoro-4-hydroxyphényl)propanoate de méthyle, le 2-(acétylamino)-2-(4-[(2-chloro-6-fluorobenzyl)oxy]benzyl-malonate de diéthyle, le (2R)-2-amino-3-(3-chloro-4-méthoxyphényl)propanoate de méthyle, le (2R)-2-amino-3-(3-chloro-4-hydroxy-5-méthoxyphényl)propanoate de méthyle, le (2R)-2-amino-3-(2,6-dichloro-3-hydroxy-4-méthoxyphényl)-propanoate de méthyle, le (2R)-2-amino-3-(3-chloro-4-hydroxyphényl)propanoate de méthyle, le H-DL-tyr-OMe HCl, le H-3,5-diiodo-tyr-OMe HCl, le H-D-3,5-diiodo-tyr-OMe HCl, le H-D-tyr-OMe HCl, le chlorhydrate d'ester méthylique de D-tyrosine, le D-tyrosine-OMe HCl, le chlorhydrate de D-tyrosinate de méthyle, l'acide (2R)-2-amino-3-(4-hydroxyphényl)propionique, le chlorhydrate d'ester méthylique de (2R)-2-amino-3-(4-hydroxyphényle), le chlorhydrate de (2R)-2-amino-3-(4-hydroxyphényl)propanoate de méthyle, le chlorhydrate de (2R)-2-azanyl-3-(4-hydroxyphényl)propanoate de méthyle, la 3-chloro-L -tyrosine, la 3-nitro-L-tyrosine, le chlorhydrate d'ester éthylique de 3-nitro-L-tyrosine, la DL-*m*-tyrosine, la DL-*o-*tyrosine, le Boc-Tyr(3,5-I₂)-OSu, le Fmoc-tyr(3-NO₂)-OH, l'α-méthyl-L-tyrosine, l'α-méthyl-D-tyrosine et l'α-méthyl-DL-tyrosine.

2. Composition destinée à être utilisée selon la revendication 1, dans laquelle ledit au moins un inhibiteur de tyrosine hydroxylase est l'α-méthyl-DL-tyrosine racémique.

3. Composition destinée à être utilisée selon l'une quelconque des revendications précédentes, dans laquelle ledit au moins un inhibiteur de tyrosine hydroxylase est formulé pour une administration orale, nasale, sous-cutanée, intraveineuse, topique, transdermique, vaginale ou rectale, ou une combinaison de celles-ci.

4. Composition destinée à être utilisée selon l'une quelconque des revendications précédentes, comprenant 50 à 1500 mg de l'au moins un inhibiteur de tyrosine hydroxylase.

5. Composition destinée à être utilisée selon l'une quelconque des revendications précédentes, dans laquelle ledit au moins un inhibiteur de la tyrosine hydroxylase est formulé en trois doses divisées.

6. Composition destinée à être utilisée selon l'une quelconque des revendications précédentes, dans laquelle ledit au moins un inhibiteur de tyrosine hydroxylase est l'α-méthyl-L-tyrosine (métyrosine) ou l'α-méthyl-D-tyrosine.

7. Composition destinée à être utilisée selon l'une quelconque des revendications précédentes, comprenant en outre un ou plusieurs modulateurs de stress, ledit modulateur de stress étant un antidépresseur, un dérivé de vasopressine, un agent neuromodulateur, un inducteur d'acétylcholine, une benzodiazépine, un glucocorticoïde ou un cannabinoïde, par exemple, dans laquelle le dérivé de vasopressine est la vasopressine ou la desmopressine ; dans laquelle l'agent neuromodulateur est le GABA, la rivastigmine ou la pilocarpine ; dans laquelle l'antidépresseur est un inhibiteur sélectif du recaptage de la sérotonine (ISRS), un inhibiteur du recaptage de la sérotonine-norépinéphrine (SNRI) ou un antidépresseur tricyclique ; ou dans laquelle l'antidépresseur est la sertraline, la fluoxétine, la paroxétine ou la venlafaxine.

8. Composition destinée à être utilisée selon la revendication 7, dans laquelle ledit dérivé de vasopressine est la 1-déamino-8-D-arginine vasopressine (DDAVP), la 1-déamino-4-valine-8-D-arginine vasopressine (DVDAVP) et la 8- arginine vasopressine (AVP).

9. Composition destinée à être utilisée selon les revendications 7 ou 8, dans laquelle ledit inhibiteur de tyrosine hydroxylase est l'α-méthyl-DL-tyrosine racémique et dans laquelle le modulateur de stress est la désompressine et le GABA.

10. Composition destinée à être utilisée selon la revendication 9, comprenant de l'α-méthyl-DL-tyrosine, de la désompressine et du GABA.

11. Composition destinée à être utilisée selon l'une quelconque des revendications précédentes, dans laquelle le traitement réduit au moins l'un des symptômes suivants : les pensées intrusives, les cauchemars, les flashbacks, la détresse émotionnelle après exposition à des rappels traumatiques, la réactivité physique après exposition à des rappels traumatiques, les pensées ou sentiments liés au traumatisme, les rappels liés au traumatisme, l'incapacité à se souvenir des caractéristiques clés du traumatisme, les pensées et hypothèses excessivement négatives sur soi-même ou sur le monde, les reproches exagérés à soi-même ou aux autres d'avoir causé le traumatisme, l'affect négatif, la diminution de l'intérêt pour les activités, le sentiment d'isolement, la difficulté à éprouver un affect positif, l'irritabilité ou l'agressivité, le comportement risqué ou destructeur, l'hypervigilance, la réaction de sursaut accrue, la difficulté à se concentrer et la difficulté à dormir.

12. Composition destinée à être utilisée selon l'une quelconque des revendications précédentes, dans laquelle ladite composition est utilisée en combinaison avec une psychothérapie, une thérapie cognitivo-comportementale, une thérapie de désensibilisation et de retraitement des mouvements oculaires (EMDR), une psychothérapie interpersonnelle, une thérapie physique, une thérapie par le jeu, ou une combinaison de celles-ci .
